# EUROPEAN PATENT SPECIFICATION

(11) **EP 2 712 625 B1**
(45) Date of publication and mention of the grant of the patent: **21.12.2016**
(21) Application number: 13178000.9
(22) Date of filing: 14.07.2009
(51) Int. Cl.: A61K 39/04, A61K 39/39, A61P 31/06

(54) **Vaccines comprising TB 10.4**
Impfstoffe mit TB 10.4
Vaccins comprenant du TB 10.4

(30) Priority: 15.07.2008 DK 200800997
(43) Date of publication of application: 02.04.2014
(62) Divisional of application: 09776173.8
(73) Proprietor: Statens Serum Institut, 2300 Copenhagen S (DK)
(72) Inventor: Dietrich, Jes, 2760 Malov (DK); Aagard, Claus, 2300 Copenhagen S (DK); Andersen, Peter, 2700 Bronshoj (DK)
(74) Representative: Plougmann Vingtoft a/s

(56) References cited:
- WO-A2-2005/061534
- "The Statens Serum Institute initiates phase I clinical trial of TB vaccine HyVac4-IC31", BIOMEDICAL LITERATURE REFERENCE FROM PROUS SCIENCE INTEGRITY , 10 December 2007 (2007-12-10), XP002547040, Retrieved from the Internet: URL:http://integrity.prous.com/integrity/s ervlet/xmlxsl/pk_ref_list.xml_show_ficha_r ef?p_ref_id=1162078 [retrieved on 2009-09-23] -& DATABASE PROUS SCIENCE INTEGRITY [Online] XP002547041, retrieved from HTTP://INTEGRITY.PROUS.COM/INTEGRITY/SERVL ET/XMLXSL/PK_PROD_LIST.EXEC_FORM_PRO_PR Database accession no. 465995
- INTERCELL AG: PRESSEPORTAL.DE , 4 December 2007 (2007-12-04), XP002511459, Retrieved from the Internet: URL:http://www.presseportal.de/pm/53037/10 96105/intercell_ag [retrieved on 2009-01-21]
- DIETRICH JES ET AL: "Exchanging ESAT6 with TB10.4 in an Ag85B fusion molecule-based tuberculosis subunit vaccine: Efficient protection and ESAT6-based sensitive monitoring of vaccine efficacy", JOURNAL OF IMMUNOLOGY, vol. 174, no. 10, 1 May 2005 (2005-05-01), pages 6332-6339, XP002440682, AMERICAN ASSOCIATION OF IMMUNOLOGISTS, US ISSN: 0022-1767
- AGGER E M ET AL: "Protective immunity to tuberculosis with Ag85B-ESAT-6 in a synthetic cationic adjuvant system IC31", VACCINE, BUTTERWORTH SCIENTIFIC, vol. 24, no. 26, 29 June 2006 (2006-06-29) , pages 5452-5460, XP025151766, GUILDFORD, GB ISSN: 0264-410X [retrieved on 2006-06-29]
- AAGAARD C ET AL: "Protection and polyfunctional T cells induced by Ag85B-TB10.4/IC31(R) against Mycobacterium tuberculosis is highly dependent on the antigen dose", PLOS ONE, vol. 4, no. 6, 16 June 2009 (2009-06-16), pages 1-8, XP002547039, PUBLIC LIBRARY OF SCIENCE, USA ISSN: 1932-6203

### SEQUENCE LISTING

<110> Statens Serum Institut
<120> Vaccines comprising TB10.4
<130> 15030pc
<160> 4
<170> PatentIn version 3.1
<210> 1
   <211> 338
   <212> PRT
   <213> Mycobacterium tuberculosis
<400> 1
   Met Gln Leu Val Asp Arg Val Arg Gly Ala Val Thr Gly Met Ser Arg
   1               5                   10                  15
   Arg Leu Val Val Gly Ala Val Gly Ala Ala Leu Val Ser Gly Leu Val
               20                  25                  30
   Gly Ala Val Gly Gly Thr Ala Thr Ala Gly Ala Phe Ser Arg Pro Gly
           35                  40                  45
   Leu Pro Val Glu Tyr Leu Gln Val Pro Ser Pro Ser Met Gly Arg Asp
       50                  55                  60
   Ile Lys Val Gln Phe Gln Ser Gly Gly Ala Asn Ser Pro Ala Leu Tyr
   65                  70                  75                  80
   Leu Leu Asp Gly Leu Arg Ala Gln Asp Asp Phe Ser Gly Trp Asp Ile
                   85                  90                  95
   Asn Thr Pro Ala Phe Glu Trp Tyr Asp Gln Ser Gly Leu Ser Val Val
               100                 105                 110
   Met Pro Val Gly Gly Gln Ser Ser Phe Tyr Ser Asp Trp Tyr Gln Pro
           115                 120                 125
   Ala Cys Gly Lys Ala Gly Cys Gln Thr Tyr Lys Trp Glu Thr Phe Leu
       130                 135                 140
   Thr Ser Glu Leu Pro Gly Trp Leu Gln Ala Asn Arg His Val Lys Pro
   145                 150                 155                 160
   Thr Gly Ser Ala Val Val Gly Leu Ser Met Ala Ala Ser Ser Ala Leu
                   165                 170                 175
   Thr Leu Ala Ile Tyr His Pro Gln Gln Phe Val Tyr Ala Gly Ala Met
               180                 185                 190
   Ser Gly Leu Leu Asp Pro Ser Gln Ala Met Gly Pro Thr Leu Ile Gly
               195                 200                 205
   Leu Ala Met Gly Asp Ala Gly Gly Tyr Lys Ala Ser Asp Met Trp Gly
       210                 215                 220
   Pro Lys Glu Asp Pro Ala Trp Gln Arg Asn Asp Pro Leu Leu Asn Val
   225                 230                 235                 240
   Gly Lys Leu Ile Ala Asn Asn Thr Arg Val Trp Val Tyr Cys Gly Asn
                   245                 250                 255
   Gly Lys Pro Ser Asp Leu Gly Gly Asn Asn Leu Pro Ala Lys Phe Leu
               260                 265                 270
   Glu Gly Phe Val Arg Thr Ser Asn Ile Lys Phe Gln Asp Ala Tyr Asn
           275                 280                 285
   Ala Gly Gly Gly His Asn Gly Val Phe Asp Phe Pro Asp Ser Gly Thr
       290                 295                 300
   His Ser Trp Glu Tyr Trp Gly Ala Gln Leu Asn Ala Met Lys Pro Asp
   305                 310                 315                 320
   Leu Gln Arg Ala Leu Gly Ala Thr Pro Asn Thr Gly Pro Ala Pro Gln
                   325                 330                 335
   Gly Ala
<210> 2
   <211> 325
   <212> PRT
   <213> Mycobacterium tuberculosis
<400> 2
   Met Thr Asp Val Ser Arg Lys Ile Arg Ala Trp Gly Arg Arg Leu Met
   1               5                   10                  15
   Ile Gly Thr Ala Ala Ala Val Val Leu Pro Gly Leu Val Gly Leu Ala
               20                  25                  30
   Gly Gly Ala Ala Thr Ala Gly Ala Phe Ser Arg Pro Gly Leu Pro Val
           35                  40                  45
   Glu Tyr Leu Gln Val Pro Ser Pro Ser Met Gly Arg Asp Ile Lys Val
       50                  55                  60
   Gln Phe Gln Ser Gly Gly Asn Asn Ser Pro Ala Val Tyr Leu Leu Asp
   65                  70                  75                  80
   Gly Leu Arg Ala Gln Asp Asp Tyr Asn Gly Trp Asp Ile Asn Thr Pro
                   85                  90                  95
   Ala Phe Glu Trp Tyr Tyr Gln Ser Gly Leu Ser Ile Val Met Pro Val
               100                 105                 110
   Gly Gly Gln Ser Ser Phe Tyr Ser Asp Trp Tyr Ser Pro Ala Cys Gly
           115                 120                 125
   Lys Ala Gly Cys Gln Thr Tyr Lys Trp Glu Thr Phe Leu Thr Ser Glu
       130                 135                 140
   Leu Pro Gln Trp Leu Ser Ala Asn Arg Ala Val Lys Pro Thr Gly Ser
   145                 150                 155                 160
   Ala Ala Ile Gly Leu Ser Met Ala Gly Ser Ser Ala Met Ile Leu Ala
                   165                 170                 175
   Ala Tyr His Pro Gln Gln Phe Ile Tyr Ala Gly Ser Leu Ser Ala Leu
               180                 185                 190
   Leu Asp Pro Ser Gln Gly Met Gly Pro Ser Leu Ile Gly Leu Ala Met
           195                 200                 205
   Gly Asp Ala Gly Gly Tyr Lys Ala Ala Asp Met Trp Gly Pro Ser Ser
       210                 215                 220
   Asp Pro Ala Trp Glu Arg Asn Asp Pro Thr Gln Gln Ile Pro Lys Leu
   225                 230                 235                 240
   Val Ala Asn Asn Thr Arg Leu Trp Val Tyr Cys Gly Asn Gly Thr Pro
                   245                 250                 255
   Asn Glu Leu Gly Gly Ala Asn Ile Pro Ala Glu Phe Leu Glu Asn Phe
               260                 265                 270
   Val Arg Ser Ser Asn Leu Lys Phe Gln Asp Ala Tyr Asn Ala Ala Gly
           275                 280                 285
   Gly His Asn Ala Val Phe Asn Phe Pro Pro Asn Gly Thr His Ser Trp
       290                 295                 300
   Glu Tyr Trp Gly Ala Gln Leu Asn Ala Met Lys Gly Asp Leu Gln Ser
   305                 310                 315                 320
   Ser Leu Gly Ala Gly
                   325
<210> 3
   <211> 340
   <212> PRT
   <213> Mycobacterium tuberculosis
<400> 3
   Met Thr Phe Phe Glu Gln Val Arg Arg Leu Arg Ser Ala Ala Thr Thr
   1               5                   10                  15
   Leu Pro Arg Arg Leu Ala Ile Ala Ala Met Gly Ala Val Leu Val Tyr
               20                  25                  30
   Gly Leu Val Gly Thr Phe Gly Gly Pro Ala Thr Ala Gly Ala Phe Ser
           35                  40                  45
   Arg Pro Gly Leu Pro Val Glu Tyr Leu Gln Val Pro Ser Ala Ser Met
       50                  55                  60
   Gly Arg Asp Ile Lys Val Gln Phe Gln Gly Gly Gly Pro His Ala Val
   65                  70                  75                  80
   Tyr Leu Leu Asp Gly Leu Arg Ala Gln Asp Asp Tyr Asn Gly Trp Asp
                   85                  90                  95
   Ile Asn Thr Pro Ala Phe Glu Glu Tyr Tyr Gln Ser Gly Leu Ser Val
               100                 105                 110
   Ile Met Pro Val Gly Gly Gln Ser Ser Phe Tyr Thr Asp Trp Tyr Gln
           115                 120                 125
   Pro Ser Gln Ser Asn Gly Gln Asn Tyr Thr Tyr Lys Trp Glu Thr Phe
       130                 135                 140
   Leu Thr Arg Glu Met Pro Ala Trp Leu Gln Ala Asn Lys Gly Val Ser
   145                 150                 155                 160
   Pro Thr Gly Asn Ala Ala Val Gly Leu Ser Met Ser Gly Gly Ser Ala
                   165                 170                 175
   Leu Ile Leu Ala Ala Tyr Tyr Pro Gln Gln Phe Pro Tyr Ala Ala Ser
               180                 185                 190
   Leu Ser Gly Phe Leu Asn Pro Ser Glu Gly Trp Trp Pro Thr Leu Ile
           195                 200                 205
   Gly Leu Ala Met Asn Asp Ser Gly Gly Tyr Asn Ala Asn Ser Met Trp
       210                 215                 220
   Gly Pro Ser Ser Asp Pro Ala Trp Lys Arg Asn Asp Pro Met Val Gln
   225                 230                 235                 240
   Ile Pro Arg Leu Val Ala Asn Asn Thr Arg Ile Trp Val Tyr Cys Gly
                   245                 250                 255
   Asn Gly Thr Pro Ser Asp Leu Gly Gly Asp Asn Ile Pro Ala Lys Phe
               260                 265                 270
   Leu Glu Gly Leu Thr Leu Arg Thr Asn Gln Thr Phe Arg Asp Thr Tyr
           275                 280                 285
   Ala Ala Asp Gly Gly Arg Asn Gly Val Phe Asn Phe Pro Pro Asn Gly
       290                 295                 300
   Thr His Ser Trp Pro Tyr Trp Asn Glu Gln Leu Val Ala Met Lys Ala
   305                 310                 315                 320
   Asp Ile Gln His Val Leu Asn Gly Ala Thr Pro Pro Ala Ala Pro Ala
                   325                 330                 335
   Ala Pro Ala Ala
               340
<210> 4
   <211> 96
   <212> PRT
   <213> Mycobacterium tuberculosis
<400> 4
   Met Ser Gln Ile Met Tyr Asn Tyr Pro Ala Met Leu Gly His Ala Gly
   1               5                   10                  15
   Asp Met Ala Gly Tyr Ala Gly Thr Leu Gln Ser Leu Gly Ala Glu Ile
               20                  25                  30
   Ala Val Glu Gln Ala Ala Leu Gln Ser Ala Trp Gln Gly Asp Thr Gly
           35                  40                  45
   Ile Thr Tyr Gln Ala Trp Gln Ala Gln Trp Asn Gln Ala Met Glu Asp
       50                  55                  60
   Leu Val Arg Ala Tyr His Ala Met Ser Ser Thr His Glu Ala Asn Thr
   65                  70                  75                  80
   Met Ala Met Met Ala Arg Asp Thr Ala Glu Ala Ala Lys Trp Gly Gly
                   85                  90                  95

## Description

### Field of the invention

The invention discloses a vaccine for human use with a low dose of an antigen comprising TB10.4 fused to a polypeptide of the antigen 85-complex e.g. Ag85B in an adjuvant and methods of immunization and treatment of *M. tuberculosis.*

### Background.

The global effort to develop a more effective *Mycobacterium tuberculosis.* (*M*. *tuberculosis)* vaccine than the currently used Bacillus of Calmette and Guerin (BCG) vaccine involves different strategies such as live attenuated vaccines (9), virally vectored *M. tuberculosis* vaccines (11), and subunit vaccines (12, 14). The subunit approach holds a number of advantages, such as increased safety and stability as well as the demonstrated ability to boost prior BCG vaccination (4, 7). In addition, as subunit vaccines appear not to be influenced by environmental mycobacteria this type of vaccine may be of particular use in the developing world (3). However, progress in this field has been delayed by the lack of adjuvants that induce a strong cell-mediated immune (CMI) response. Therefore, there still remains a need for an immunogenic composition which can generate polyfunctional immune cells thereby providing greater protection against *M. tuberculosis.*

The initiation of a phase I clinical trial of HyVac4-IC31® as well as its composition is disclosed in XP002547040, URL:http://integrity.prous.com/integrity/servlet/xmlxsl/pk_ref_list.xml_show_ficha_ref?
p_ref_id=1162078", dated 10 December 2007, entitled "The Statens Serum Institute initiates phase I clinical trial of TB vaccine HyVac4-IC31", and in Intercell AG, 4 December 2007, XP002511459, URL:http://www.presseportal.de/
pm/53037/1096105/intercell_ag.

Dietrich et al. (2005) J. Immunol. vol. 174, no. 10, pages 6332-6339, (8), is entitled "Exchanging ESAT6 with TB10.4 in an Ag85B fusion molecule-based tuberculosis subunit vaccine: Efficient protection and ESAT6-based sensitive monitoring of vaccine efficacy".

WO 2005/061534 A2 discloses improved tuberculosis vaccines that may comprise an Ag85B-TB10.4 fusion polypeptide and an adjuvant.

Agger et al. (2006) Vaccine vol. 24, no. 26, pages 5452-5460, (1), entitled "Protective immunity to tuberculosis with Ag85B-ESAT-6 in a synthetic cationic adjuvant system IC31", discloses a study of the combination of Ag85B-ESAT-6 and IC31® in a mouse aerosol challenge model of tuberculosis.

Aagaard et al. (June 2009) Plos One, vol. 4, no. 6, pages 1-8, entitled "Protection and poly-functional T cells induced by Ag85B-TB10.4/IC31(R) against Mycobacterium tuberculosis is highly dependent on the antigen dose", is a scientific publication related to the present invention.

### Summary

The present work examined the combination of Ag85B-TB10.4 (Hyvac 4) and the IC31® adjuvant as a new vaccine against infection with *M. tuberculosis.* The results show that Ag85B-TB10.4 and the IC31® adjuvant induces high amounts of polyfunctional CD4⁺ T cells and provides significant protection against *M. tuberculosis.* Surprisingly, the combination of the Ag85B-TB10.4 antigen and the IC31® adjuvant was sensitive to the antigen dose. Thus, whereas a standard dose in mice of 5.0µg of Ag85B-TB10.4 in IC31® did not lead to protection against *M. tuberculosis,* 0.5µg Ag85B-TB10.4 in IC31® induced protection comparable to that of BCG.

### Detailed description

The present invention relates to an immunogenic composition for human use comprising:
a) a fusion protein of the TB 10.4 protein and an Ag85-complex protein, and
b) an adjuvant comprising at least one polycationic peptide and at least one oligonucleotide which is oligo dIC,
wherein the Ag85-complex protein is an Ag85B protein, the total amount of the TB 10.4 protein and the Ag85B protein ranges from 5.0 to 25.0 µg, and the adjuvant is a mixture of NH₂-KLKLLLLLKLK-COOH and 5'-ICI CIC ICI CIC ICI CIC ICI CIC IC-3', for use as a vaccine against *M. tuberculosis* in a human.

In one embodiment, the fusion protein is present in the immunogenic composition in an amount of less than about 10.0 µg.

The disclosed immunogenic composition comprises an adjuvant. The preferred adjuvant of the invention is one having at least one polycationic peptide and at least one oligonucleotide; preferably the oligonucleotide is a TLR9 agonist.

The preferred adjuvant of the invention is IC31® and the preferred protein from the Ag85-complex is an Ag85B protein.

The invention discloses a vaccine for human use comprising the above mentioned immunogenic composition.

In one embodiment, the total amount of the TB10.4 protein and the Ag85-complex protein in the vaccine is less than about 10.0 µg.

The invention further discloses a method of inducing protection against *M. tuberculosis* in a human, the method comprising introducing into the human an immunogenic composition as described above.

The present work examined the combination of an Ag85B-TB10.4 (Hyvac 4) fusion protein and IC31® as a new vaccine against infection with *M. tuberculosis.* The IC31® adjuvant comprises cationic peptides and a TLR9 receptor agonist.

In an effort to generate an efficient vaccine against infection with *M. tuberculosis,* the combination of the Ag85B-TB10.4 fusion protein and IC31® is attractive for the following reasons:
(1) IC31® can promote a strong Th1 response;
(2) IC31® has a promising profile in clinical trials; it has successfully been tested in clinical phase 1 trial in combination with a trivalent influenza vaccine as well as in combination with a tuberculosis vaccine (50 ug Ag85B-ESAT-6 in IC31®; clinical data not published yet). Animal studies with Ag85B-ESAT6 in IC31® are described in (1);
(3) Ag85B-TB10.4 fusion protein has the advantage that it does not include any of the proteins that are useful for diagnostic purposes such as e.g. ESAT-6. ESAT-6 is an extremely valuable diagnostic reagent and the basis of a number of commercial diagnostic tests (5, 10, 13). Leaving ESAT-6 out of a future vaccine will allow the diagnostic tests and the vaccine to be used in parallel since the Ag85B-TB10.4 fusion protein does not compromise any of the specific diagnostic tests;
(4) Since both Ag85B and TB10.4 are expressed by BCG, the vaccine of the current invention may also be employed as a BCG booster vaccine.

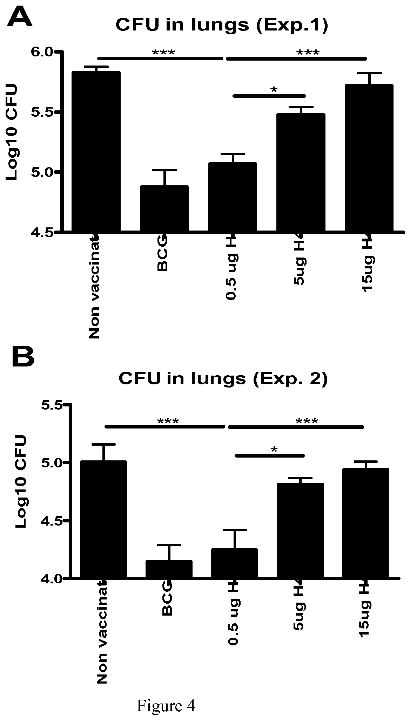

The results show that the Ag85B-TB10.4 and IC31® combination induces a high amount of polyfunctional CD4⁺T cells and provides significant protection against *M. tuberculosis.* Surprisingly, the combination of the Ag85B-TB10.4 fusion protein and the IC31® adjuvant is extremely sensitive to the antigen dose. Thus, whereas a dose of 5.0µg of Ag85B-TB10.4 in IC31® does not lead to significant protection against *M. tuberculosis*, 0.5µg Ag85B-TB10.4 in IC31® induces a strong protection comparable to that of BCG (Ex.3 & figure 4). In contrast, previous work with other antigens, such as Ag85B-ESAT6 in MPL-DDA showed that the optimal antigen dose for this fusion protein was approximately 10.0µg (16) and that a dose of 5µg of Ag85B-TB10.4 in MPL-DDA provided significant protection comparable to BCG (8) indicating that Ag85B-TB10.4 is an extraordinary immunogenic molecule.

This is the first study to show that the Ag85B-TB10.4 fusion protein in the IC31® adjuvant constitutes an effective vaccine against infection with *M. tuberculosis.*

Throughout this specification, unless the context requires otherwise, the word "comprise", or variations thereof such as "comprises" or "comprising", will be understood to imply the inclusion of a stated element or integer or group of elements or integers but not the exclusion of any other element or integer or group of elements or integers.

### Antigens

The antigen of the present invention comprises a protein of the Ag85-complex fused to TB10.4 protein, including, for example, proteins Ag85A, Ag85B or Ag85C of the Ag85 complex.

Ag85B-TB10.4 is an extraordinary immunogenic molecule which must be given in low doses to initiate the maximum amount of polyfunctional immune cells inducing more interferon-γ expression and increased protection against *M. tuberculoses.* Throughout this specification Ag85B-TB10.4 fusion protein is interchangeable with the terms H4 and HyVac4.

Also described herein is an embodiment in which the antigen may comprise a protein of the Ag85 complex and TB10.4 protein wherein the Ag85 complex protein is not fused to the TB10.4 protein.
The preferred protein of the Ag85 complex is Ag85B.

| Protein | amino acid sequence |
|---|---|
| Ag85A | |
| SEQ ID NO. 1 | |
| Ag85B | |
| SEQ ID NO. 2 | |
| | |
| Ag85C | |
| SEQ ID NO. 3 | |
| TB10.4 | |
| SEQ ID NO. 4 | |

Each protein may be modified by glycosylation, or lipidation (Mowat et al. 1991 ;Lustig et al. 1976). Each protein may be modified by the addition of prosthetic groups, a purification moiety, or a signal peptide. Each protein may be modified one or more times or not undergo any modification. Each protein may be modified singly or in combination. Each protein will be characterised by specific amino acids and be encoded by specific nucleic acid sequences. Within the scope of the present invention are such sequence and analogues and variants produced by recombinant or synthetic methods wherein such amino acid sequences have been modified by substitution, insertion, addition or deletion of one or more amino acid residues in the recombinant protein while retaining immunogenicity as confirmed by any one or all of the biological assays described herein.

Substitutions are preferably "conservative". These are defined according to the following table. Amino acids in the same block in the second column and preferably in the same line in the third column may be substituted for each other. The amino acids in the third column are indicated in one-letter code.

| | | |
|---|---|---|
| ALIPHATIC | Non-polar | GAP |
| | | ILV |
| | Polar-uncharged | CSTM |
| | | NQ |
| | Polar-charged | DE |
| | | KR |
| AROMATIC | | HFWY |

### Adjuvants

In the present invention the antigen comprises an Ag85 complex protein fused to TB10.4 protein in an adjuvant.

Also described herein is an embodiment in which the antigen may comprise an Ag85 complex protein and a TB10.4 protein (i.e. non-fused) in an adjuvant.

Ag85B-TB10.4 is an extraordinary immunogenic molecule which must be given in low doses to initiate the maximum amount of polyfunctional immune cells inducing more interferon-γ expression and increased protection against *M .tuberculoses.* It is exemplified with an adjuvant comprising a polycationic peptide [e.g. polylysin (KLK peptide) or polyarginin] and oligodeoxynucleic molecules but is not limited to this adjuvant.

The preferred adjuvant of the present invention is a mixture of a polycationic peptide [e.g. polylysin (KLK peptide) or polyarginin] and oligodeoxynucleic molecules [I-ODNs]. An example of a suitable I-ODN for use with the current invention is oligo dIC e.g. 5'- ICI CIC ICI CIC ICI CIC ICI CIC IC-3'. A thorough review I-ODN's appropriate for use with the present invention is provided in Patent Cooperation Treaty International Application Publication No. WO0193905 and WO0193903. Examples of suitable KLK peptides are NH2KLKLLLLLKLK-COOH or KLKLLLLLKLK-NH2. Additional examples of appropriate polycationic peptides are RLRLLLLLRLR-NH2, RLKLLLLLKLR-NH2, KFKFFFFFKFK-NH2, KWKWWWWWKWK-NH2 or KVKVVVVVKVK-NH2. A thorough review of the polycationic peptides suitable for use with the current invention is provided in Patent Cooperation Treaty International Application Publication No. WO0232451.

The IC31® adjuvant comprises a mixture of the peptide NH₂-KLKLLLLLKLK-COOH (obtained from Multiple Peptide Systems, San Diego, CA, USA) and the oligonucleotide 5'-ICI CIC ICI CIC ICI CIC ICI CIC IC-3' (purchased from Proligo, Boulder, USA).

Previous studies have shown that the Ag85B-TB10.4 fusion protein or Ag85B-ESAT-6 fusion protein in cationic liposomes induces efficient protection against *M. tuberculosis* and that Ag85B-ESAT-6 in IC31® is as protective as BCG in a mouse *M. tuberculosis* animal model (1, 8, 12). In each of these studies a standard antigen dose of 5.0µg was employed.

In the current study, a dose of 0.5µg Ag85B-TB10.4 fusion protein in IC31 [100 nmol peptide (NH₂-KLKLLLLLKLK-COOH) and 5 nmol oligonucleotide (5'- ICI CIC ICI CIC ICI CIC ICI CIC IC-3')] induced a strong INF-γ response. This strong INF-γ response was in approximately the same range as 5.0µg Ag85B-TB10.4 fusion protein, without IC31. Moreover, 0.5µg Ag85B-TB10.4 fusion protein in IC31 [100 nmol peptide (NH₂-KLKLLLLLKLK-COOH) and 5 nmol oligonucleotide (5'- ICI CIC ICI CIC ICI CIC ICI CIC IC-3')] provided a strong protection in approximately the same range as BCG. This is surprisingly better than 5.0µg or 15.0µg of Ag85B-TB10.4 fusion protein alone (i.e. no IC31), revealing that the optimal dose of Ag85B-TB10.4 fusion protein alone is less than about 5.0µg per dose.

Moreover, the lowest dose of Ag85B-TB10.4 fusion protein (0.5µg) in IC31 gave the highest IFN-γ response after stimulation with either of the vaccine components (see Figures 1 and 2). Finally, vaccination with Ag85B-TB10.4 fusion protein in IC31 induced two major CD4⁺T cell populations, one expressing IFN-γ, IL-2, and TNF-α, and another expressing IL-2 and TNF-α. Both of these T cell populations belong to central memory T cells, and are necessary for long term memory (17). Importantly, as seen with the IFN-γ expression measured by ELISA, using a dose of 0.5µg HyVac4 fusion protein in IC31 induced the highest cell numbers within the polyfunctional population that expressed IFN-γ, IL-2, and TNF-α, (see Figure 3).

The present invention demonstrates the following:
(1) the adjuvants IC31 and cationic liposomes exhibit different sensitivities towards the antigen dose; and
(2) the optimal antigen dose in IC31 is antigen-dependent.
For example, 0.5µg Ag85B-TB10.4 fusion protein in IC31 induced significant protection whereas 5.0µg Ag85B-TB10.4 fusion protein in IC31 does not (figure 4).
Taken together, these results highlight the importance of analyzing the antigen dose when testing new adjuvant/antigen formulations and teach that the optimal antigen dose in a vaccine depends both on the antigen and on the adjuvant.

Moreover, in the present invention, the observed correlation between the amount of poly-functional T cells and the strength of protection induced by the Ag85B-TB10.4 vaccine in IC31 is in agreement with a recent study in which protective immunity against infection with *Leishmania major* was directly correlated with long lived memory response and the number of polyfunctional T cells generated (6).

### Figure Legends.

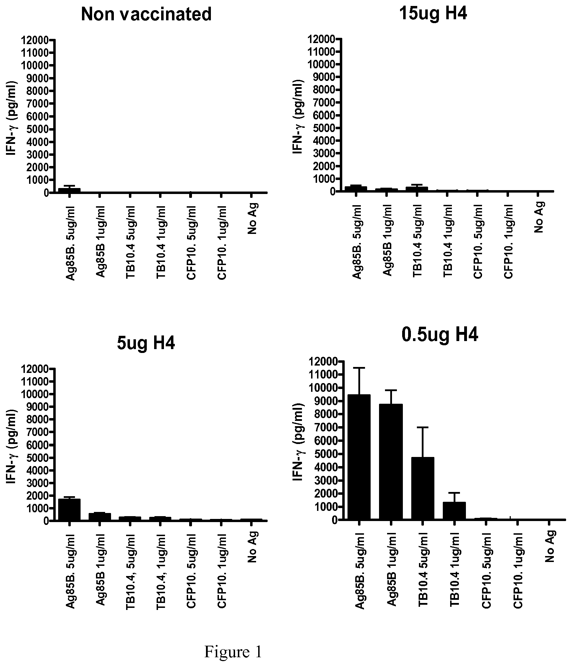

**Figure 1****.** Immune recognition of vaccination antigens. PBMC's isolated from groups of mice vaccinated with different doses of H4 in IC31 and a saline control group were stimulated with either 1.0 or 5.0µg/ml of Ag85B, TB10.4 or CFP10. After 72 hours the concentration of cell released IFN-γ was determined by ELISA. PBMC's were isolated 1 week after the third vaccination and were pooled from five mice per group. Values represent the means of triplicate and SEM's are indicated by bars.

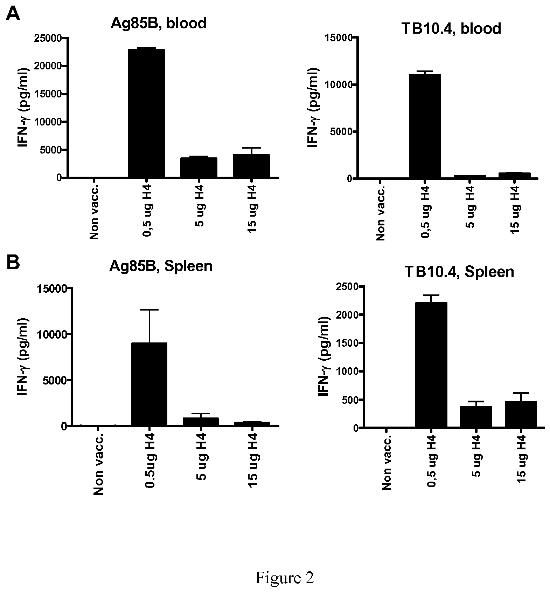

**Figure 2****.** Recognition of Ag85B in different organs. PBMC's (A) and spleenocytes (B) isolated from groups of mice immunized with 3 different doses of H4 formulated in IC31 or a saline control group were stimulated with Ag85B or TB10.4 for 72 hours whereafter IFN-γ cytokine secretion was measured by ELISA. The bars represent means of 3 individual mice. SEMs are indicated. In both (A) and (B) a vaccination dose of 0.5µg H4 gave significantly (p < 0.05, one-way ANOVA and Tukey's post test) higher antigen responses, compared to vaccination doses of 5.0µg and 15.0µg.

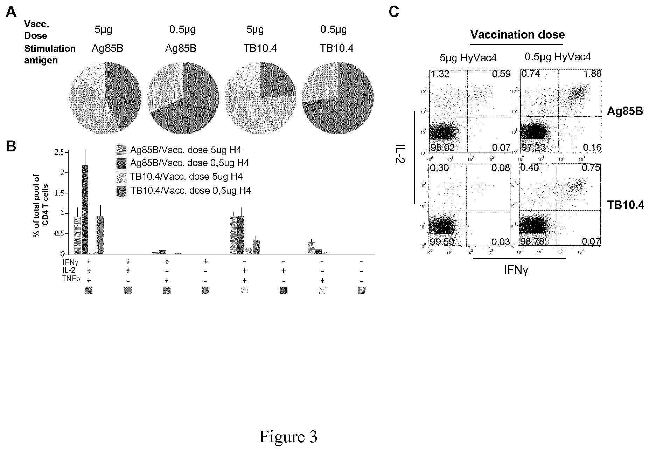

**Figure 3****.** Ag85B and TB10.4 specific T cells are poly-functional. (A) Production of IFN-γ, TNF-α and IL-2 was assessed following antigenic stimulation of PBMC's and spleenocytes 2 weeks post vaccination by flow cytometry. The pie charts are grouped after vaccination dose and colour coded according to the cytokine production profile and summarize the fractions of the CD4⁺ T cell response that are positive for a given cytokine production profile. (B) Every possible combination of cytokines is shown on the x-axis of the bar chart and the percentage of Hyvac4 (H4) specific CD4⁺ T cells expressing any combination of cytokines is given for each immunization group. No responses were seen in the CD8⁺ T cell subset. (C) Dot plots from the FACS analysis in (A) showing IL-2 and IFN-γ expression by CD4⁺ spleen cells from mice vaccinated with 0.5µg or 5.0µg HyVac4. Percentages in each quadrant are indicated.
**Figure 4****.** Protective efficacy of H4. In two independent experiments (A and B) groups of mice were vaccinated with three different doses of H4 formulated in IC31 and compared to saline and BCG-vaccinated controls. All groups were challenged by the aerosol route with virulent *M. tuberculosis* ten weeks after the first vaccination. Six weeks post-challenge, all mice were killed and the bacterial burden (CFU) was measured in the lung by bacterial count. In both experiments data are presented as mean values from six animals per group and standard errors of the means are indicated by bars. Statistical comparison among the vaccination groups was done by one-way ANOVA and Tukey's post test. Significant differences are only shown for selected groups. ***: p < 0.001, *: p < 0.05.

### Materials and Methods

### Animals.

Studies were performed with 8 to 12 week-old C57BL/6xBalb/c F1 female mice, purchased from Taconic, Ejby, Denmark. Infected animals were housed in cages contained within laminar flow safety enclosures in a BSL-3 facility. The use of mice was done in accordance with the regulations set forward by the Danish Ministry of Justice and Animal Protection Committees and in compliance with EC Directive 86/609 and the US ALAC recommendations for the care and use of Laboratory animals.

### Bacteria.

*M. tuberculosis.* Erdman were grown at 37°C on Lowenstein-Jensen medium or in suspension in Sauton medium enriched with 0.5% sodium pyruvate and 0.5% glucose.

### Immunization.

Mice were immunized three times at 2-week intervals subcutaneously on the back with experimental vaccines containing 0.5, 5.0 or 15.0µg of Ag85B-TB10.4 fusion protein (H4)/dose, emulsified in IC31 in a total volume of 0.2 ml/dose. Doses were 100 nmol peptide and 5 nmol oligonucleotide. All vaccines were formulated using 10 mM Tris-HCL/270 mM sorbitol buffer (pH 7.9) as previously described [12] to obtain a final volume of 0.2 ml/mouse. At the time of the first subunit vaccination, one group of mice received a single dose of BCG Danish 1331 (2.5 x 10⁵ CFU) injected subcutaneously at the base of the tail and one group received a saline injection. All groups of mice were challenged 10 weeks after the first vaccination.

### Experimental infections

When challenged by the aerosol route, the animals were infected with approximately 50 CFU of *M. tuberculosis.* Erdman/mouse. These mice were sacrificed 6 weeks after challenge. Numbers of bacteria in the spleen or lung were determined by serial threefold dilutions of individual whole-organ homogenates in duplicate on 7H11 medium. Organs from the BCG-vaccinated animals were grown on medium supplemented with 2.0µg of 2-thiophene-carboxylic acid hydrazide (TCH)/ml to selectively inhibit the growth of the residual BCG bacteria in the test organs. Colonies were counted after 2 to 3 weeks of incubation at 37°C. Bacterial burden in the lungs was expressed as log10 of the bacterial counts based on vaccination groups of six animals.

### Lymphocyte cultures

Lymphocytes from spleens were obtained as described previously (2). Blood lymphocytes (PBMCs) were purified on a density gradient. Cells pooled from five mice in each experiment were cultured in microtiter wells (96-well plates; Nunc, Roskilde, Denmark) containing 2 x 10⁵ cells in a volume of 200µl of RPMI 1640 supplemented with 5 x 10⁻⁵ M 2-mercaptoethanol, 1% penicillin-streptomycin, 1 mM glutamine, and 5% (vol/vol) fetal calf serum. Based on previous dose-response investigations, the mycobacterial antigens were all used at 15µg/ml or 5µg/ml, while concanavalin A was used at a concentration of 1µg/ml as a positive control for cell viability. All preparations were tested in cell cultures and found to be nontoxic at the concentrations used in the present study. Supernatants were harvested from cultures after 72h of incubation for the investigation of IFN-γ.

### IFN-γ enzyme-linked immunosorbent assay (ELISA).

Microtiter plates (96 wells; Maxisorb; Nunc) were coated with monoclonal hamster anti-murine IFN-γ (Genzyme, Cambridge, Mass.) in PBS at 4°C. Free binding sites were blocked with 1% (wt/vol) bovine serum albumin-0.05% Tween 20. Culture supernatants were tested in triplicate, and IFN-γ was detected with a biotin-labelled rat anti-murine monoclonal antibody (clone XMG1.2; Pharmingen, San Diego, CA). Recombinant IFN-γ (Pharmingen, San Diego, CA) was used as a standard.

### FACS analysis of lymphocytes.

Intracellular cytokine staining procedure: Cells from blood, spleen or lungs of mice were stimulated for 1-2 h with 2µg/ml Ag and subsequently incubated for 6 h with 10µg/ml brefeldin A (Sigma-Aldrich, USA) at 37°C. Thereafter, cells were stored overnight at 4° C. The following day, Fc receptors were blocked with 0.5µg/ml anti-CD16/CD32 mAb (BD Pharmingen, USA) for 10 minutes, where after the cells were washed in FACS buffer (PBS containing 0.1% sodium azide and 1% FCS), and stained for surface markers as indicated using 0.2µg/ml anti-CD4 (clone: RM4-5), anti-CD8 (clone: 53-6,7) mAb's. Cells were then washed in FACS buffer, permeabilized using the Cytofix/Cytoperm kit (BD Pharmingen, Denmark) according to the manufacturers instructions, and stained intracellularly with 0.2 µg/ml anti-IFN- y (clone: XMG1.2), anti-TNF-α (clone: MP6-XT22), or anti-IL-2 (clone: JES6-5H4) mAb's. After washing, cells were re-suspended in formaldehyde solution 4% (w/v) pH 7.0 (Bie & Berntsen, Denmark) and analysed by flow cytometry on a six-colour BD FACSCanto flow cytometer (BD Biosciences, USA).

### Statistical methods

The data obtained were tested by analysis of variance. Differences between means were assessed for statistical significance by Tukey's test. A P value of < 0.05 was considered significant.

### Example 1

### Immune response induced after immunization with Ag85B-TB10.4 fusion protein in IC31

We first analyzed the immunogenicity of Ag85B-TB10.4 fusion protein delivered in IC31 and whether both components of the fusion protein were recognized by the immune system after immunization. Groups of mice were immunized with Ag85B-TB10.4 fusion protein in IC31. As negative control, a group of mice received the adjuvant alone (data not shown). To examine the optimal antigen dose in IC31, we used 15.0, 5.0 and 0.5µg of Ag85B-TB10.4 fusion protein. One week after the last injection, mice were bled, and the IFN-γ release was evaluated after *in vitro* stimulation of purified PBMCs with different concentrations of Ag85B and TB10.4 proteins (5µg/ml and 1µg/ml) (Fig. 1A). Immunization with Ag85B-TB10.4 fusion protein in IC31 induced a strong IFN-γ response specific for Ag85B and TB10.4 proteins. Surprisingly, this response was sensitive to the antigen immunization dose. Thus, the lowest dose of Ag85B-TB10.4 fusion protein in IC31 gave the highest IFN-γ response after stimulation with either Ag85B (9401 +/- 3668 pg/ml IFN-γ) or TB10.4 (4694 +/- 3992 pg/ml IFN-γ) (Fig. 1A). Using a dose of 5.0µg or 15.0µg Ag85B-TB10.4 fusion protein significantly reduced the IFN-γ response against both Ag85B and TB10.4 proteins relative to mice vaccinated with 0.5µg Ag85B-TB10.4 fusion protein (p<0.001). This was particularly apparent for the high immunization dose - 15µg Ag85B-TB10.4 fusion protein per immunization dose - which gave IFN-γ responses that did not differ from the observed responses in non-vaccinated mice (or in Ag85B-TB10.4 fusion protein vaccinated mice stimulated in vitro with control antigen CFP10). The same dose dependency was subsequently repeated in an independent experiment where the immune responses were analyzed in both blood and spleen (Fig 2). These results show that the lowest dose of 0.5µg Ag85B-TB10.4 fusion protein in IC31 induced the strongest systemic response of the antigen doses tested.

### Example 2

### Vaccination with Ag85B-TB10.4 fusion protein in IC31 induces polyfunctional CD4⁺T cells.

We next analyzed the phenotype of the T cells induced by immunizing with Ag85B-TB10.4 fusion protein in IC31. In particular, we were interested in the ability of this vaccine to induce polyfunctional (IFN-γ⁺IL-2⁺TNF-α⁺) CD4⁺ T cells as these have been shown to correlate with protective immunity against infections such as *Leishmania major* and to form the basis for a long lived memory response (6, 15). PBMC's from Ag85B-TB10.4 fusion protein in IC31 vaccinated mice were stimulated in vitro with Ag85B or TB10.4 fusion protein and analyzed by flow cytometry for expression of CD4, CD8, IFN-γ, TNF- *α*, and IL-2. The results showed that immunizing with Ag85B-TB10.4 fusion protein in IC31 induced two major polyfunctional T cell populations; CD4⁺IFN-γ⁺IL-2⁺TNF-α⁺ and CD4⁺IL-2⁺TNF-α⁺ T cells (Fig. 3). This was seen for Ag85B and TB10.4 specific T cells. Interestingly, as observed in figure 1, there is a higher response in the group immunized with 0.5µg HyVac4 in IC31 compared to the group immunized with 5.0µg HyVac4 fusion protein in IC31 (see Figure 3), and that the major difference was that the group being vaccinated with only 0.5µg showed significantly more polyfunctional T cells. Taken together, immunizing with Ag85B-TB10.4 fusion protein in IC31 induced poly-functional CD4⁺ T cells and confirmed that lowering the amount of Ag85B-TB10.4 fusion protein increased the immunogenicity of the vaccine in terms of not only IFN-γ expression but also the number of polyfunctional T cells.

### Example 3

### Protective efficacy of Ag85B-TB10.4 fusion protein and IC31 in a mouse M. tuberculosis infection model

We finally examined the protective efficacy of Ag85B-TB10.4 fusion protein in IC31, and whether the dose dependency regarding the immunogenicity of the vaccine was also reflected in the protective efficacy of the vaccine. Mice were vaccinated three times at two week intervals with Ag85B-TB10.4 fusion protein in IC31. As a positive control for protection, a group of mice were immunized once with BCG. Ten weeks after the first vaccination, the mice were challenged by the aerosol route with virulent *M. tuberculosis* Erdman. Six weeks post challenge, the mice were killed and the bacterial numbers were determined in the lungs. As observed with the immunogenicity of the vaccines, the lowest Ag85B-TB10.4 fusion protein immunization dose induced the highest protection. Thus, mice vaccinated with 0.5µg Ag85B-TB10.4 fusion protein in IC31 contained a bacterial number of 5.0 +/- 0.2 Log₁₀ CFU in the lungs. This was equal to the numbers observed in BCG vaccinated mice (4.90 +/- 0.35 Log₁₀ CFU), but significantly lower (p<0.001) compared to the bacterial numbers in non-vaccinated mice (5.83 +/- 0.12 Log₁₀ CFU) (Fig. 4A). In contrast, the bacterial numbers in mice vaccinated with 5.0µg or 15.0µg of Ag85B-TB10.4 fusion protein in IC31, were not significantly different from the levels found in the lungs of non-vaccinated mice (Fig. 4A). Repeating the experiment led to the same conclusion although the overall bacterial numbers were slightly lower in all the groups (Fig. 4B).Thus the ability of the vaccine, Ag85B-TB10.4 fusion protein in IC31, to induce protection against *M. tuberculosis* correlated with the immunogenicity of the vaccine, in terms of INF-γ production and the number of polyfunctional T cells, and was highest when the lowest antigen dose was used.

The surprising *in vivo* results from these well recognized *M. tuberculosis* animal models, support the use of the immunogenic compositions of the current invention as an *M. tuberculosis* vaccine in humans.

### Example 4

### Protective efficacy of Ag85B-TB10.4 fusion protein and IC31 in a clinical trial

In human clinical trials subjects will be vaccinated with less than about 5.0µg to 25.0µg of Ag85B-TB10.4 in IC31.
This low dose of Ag85B-TB10.4 is in stark contrast with other subunit *M. tuberculosis* vaccines currently in clinical trials. For example, 40.0µg of MTB72F in AS02A per dose (19) and 50.0µg of Ag85B-ESAT-6 in IC31 per dose (clinical data not published yet).

The above disclosure generally describes the present invention. A more complete understanding can be obtained by reference to the following specific Examples. These Examples are described solely for purposes of illustration and are not intended to limit the scope of the invention.

### References.

1. Agger, E. M., I. Rosenkrands, A. W. Olsen, G. Hatch, A. Williams, C. Kritsch, K. Lingnau, A. von Gabain, C. S. Andersen, K. S. Korsholm, and P. Andersen. 2006. Protective immunity to tuberculosis with Ag85B-ESAT-6 in a synthetic cationic adjuvant system IC31. Vaccine 24:5452-5460.
2. Brandt, L., M. Elhay, I. Rosenkrands, E. B. Lindblad, and P. Andersen. 2000. ESAT-6 subunit vaccination against Mycobacterium tuberculosis. Infect Immun 68:791-795.
3. Brandt, L., J. Feino Cunha, A. Weinreich Olsen, B. Chilima, P. Hirsch, R. Appelberg, and P. Andersen. 2002. Failure of the Mycobacterium bovis BCG vaccine: some species of environmental mycobacteria block multiplication of BCG and induction of protective immunity to tuberculosis. Infect Immun 70:672-678.
4. Brandt, L., Y. A. Skeiky, M. R. Alderson, Y. Lobet, W. Dalemans, O. C. Turner, R. J. Basaraba, A. A. Izzo, T. M. Lasco, P. L. Chapman, S. G. Reed, and I. M. Orme. 2004. The protective effect of the Mycobacterium bovis BCG vaccine is increased by coadministration with the Mycobacterium tuberculosis 72-kilodalton fusion polyprotein Mtb72F in M. tuberculosis-infected guinea pigs. Infect Immun 72:6622-6632.
5. Brock, I., K. Weldingh, E. M. Leyten, S. M. Arend, P. Ravn, and P. Andersen. 2004. Specific T-cell epitopes for immunoassay-based diagnosis of Mycobacterium tuberculosis infection. J Clin Microbiol 42:2379-2387.
6. Darrah, P. A., D. T. Patel, P. M. De Luca, R. W. Lindsay, D. F. Davey, B. J. Flynn, S. T. Hoff, P. Andersen, S. G. Reed, S. L. Morris, M. Roederer, and R. A. Seder. 2007. Multifunctional TH1 cells define a correlate of vaccine-mediated protection against Leishmania major. Nat Med 13:843-850.
7. Dietrich, J., C. Andersen, R. Rappuoli, T. M. Doherty, C. G. Jensen, and P. Andersen. 2006. Mucosal administration of Ag85B-ESAT-6 protects against infection with Mycobacterium tuberculosis and boosts prior bacillus Calmette-Guerin immunity. J Immunol 177:6353-6360.
8. Dietrich, J., C. Aagaard, R. Leah, A. W. Olsen, A. Stryhn, T. M. Doherty, and P. Andersen. 2005. Exchanging ESAT6 with TB10.4 in an Ag85B fusion molecule-based tuberculosis subunit vaccine: efficient protection and ESAT6-based sensitive monitoring of vaccine efficacy. J Immunol 174:6332-6339.
9. Horwitz, M. A., G. Harth, B. J. Dillon, and S. Maslesa-Galic. 2000. Recombinant bacillus calmette-guerin (BCG) vaccines expressing the Mycobacterium tuberculosis 30-kDa major secretory protein induce greater protective immunity against tuberculosis than conventional BCG vaccines in a highly susceptible animal model. Proc Natl Acad Sci U S A 97:13853-13858.
10. Lalvani, A., A. A. Pathan, H. McShane, R. J. Wilkinson, M. Latif, C. P. Conlon, G. Pasvol, and A. V. Hill. 2001. Rapid detection of Mycobacterium tuberculosis infection by enumeration of antigen-specific T cells. Am J Respir Crit Care Med 163:824-828.
11. Lustig JV, Rieger HL, Kraft SC, Hunter R, Rothberg RM. 1976, Cell Immunol 24(1):164-7
12. McShane, H., A. A. Pathan, C. R. Sander, S. M. Keating, S. C. Gilbert, K. Huygen, H. A. Fletcher, and A. V. Hill. 2004. Recombinant modified vaccinia virus Ankara expressing antigen 85A boosts BCG-primed and naturally acquired antimycobacterial immunity in humans. Nat Med 10:1240-1244.
13. Mowat AM, Donachie AM, Reid G, Jarrett O. 1991, Immunology 72(3):317-22
14. Olsen, A. W., L. A. van Pinxteren, L. M. Okkels, P. B. Rasmussen, and P. Andersen. 2001. Protection of mice with a tuberculosis subunit vaccine based on a fusion protein of antigen 85b and esat-6. Infection and Immunity 69:2773-2778.
15. Ravn, P., A. Demissie, T. Eguale, H. Wondwosson, D. Lein, H. Amoudy, A. S. Mustafa, A. K. Jensen, A. Holm, I. Rosenkrands, F. Oftung, J. Olobo, C. F. von-Reyn, and P. Andersen. 1999. Human T cell responses to the ESAT-6 antigen from Mycobacterium tuberculosis. J.Infect.Dis. 179:637-645.
16. Skeiky, Y. A., M. R. Alderson, P. J. Ovendale, J. A. Guderian, L. Brandt, D. C. Dillon, A. Campos-Neto, Y. Lobet, W. Dalemans, I. M. Orme, and S. G. Reed. 2004. Differential immune responses and protective efficacy induced by components of a tuberculosis polyprotein vaccine, Mtb72F, delivered as naked DNA or recombinant protein. J Immunol 172:7618-7628.
17. Stockinger, B., C. Bourgeois, and G. Kassiotis. 2006. CD4+ memory T cells: functional differentiation and homeostasis. Immunol Rev 211:39-48.
18. Weinreich Olsen, A., L. A. van Pinxteren, L. Meng Okkels, P. Birk Rasmussen, and P. Andersen. 2001. Protection of mice with a tuberculosis subunit vaccine based on a fusion protein of antigen 85b and esat-6. Infect Immun 69:2773-2778.
19. Wu, C. Y., J. R. Kirman, M. J. Rotte, D. F. Davey, S. P. Perfetto, E. G. Rhee, B. L. Freidag, B. J. Hill, D. C. Douek, and R. A. Seder. 2002. Distinct lineages of T(H)1 cells have differential capacities for memory cell generation in vivo. Nature immunology 3:852-858.
20. US FDA. 1995. Guidance for industry: Estimating the Maximum Safe Starting Dose in initial clinical trials for therapeutics in adult healthy volunteers. U.S. Department of Health and Human Services, Food and Drug Administration, Center for Drug Evaluation and Research.
21. Leroux-Roels I, Leroux-Roels G, Ofori-Anyinam O et al. Safety and immunogenicity of the Mtb72f/AS02A tuberculosis vaccine in PPD-negative Belgian adults. Medical and Health in the Tropics. Marseille, France, 11-15 Sept 2005 (Abstract O-036)

## Claims

1. An immunogenic composition comprising:
a) a fusion protein of the TB 10.4 protein and an Ag85-complex protein, and
b) an adjuvant comprising at least one polycationic peptide and at least one oligonucleotide which is oligo dIC,
wherein the Ag85-complex protein is an Ag85B protein, the total amount of the TB 10.4 protein and the Ag85B protein ranges from 5.0 to 25.0 µg, and the adjuvant is a mixture of NH₂-KLKLLLLLKLK-COOH and 5'-ICI CIC ICI CIC ICI CIC ICI CIC IC-3',
for use as a vaccine against *M. tuberculosis* in a human.

## Patentansprüche

1. Immunogene Zusammensetzung, umfassend:
a) ein Fusionsprotein des TB 10.4 Proteins und eines Ag85-Komplex-Proteins und
b) ein Adjuvans, umfassend mindestens ein polykationisches Peptid und mindestens ein Oligonukleotid, das ein Oligo-dIC ist,
wobei das Ag85-Komplex-Protein ein Ag85B Protein ist, die Gesamtmenge von TB 10.4 Protein und Ag85B Protein im Bereich von 5,0 bis 25,0 µg liegt und das Adjuvans eine Mischung aus NH₂-KLKLLLLLKLK-COOH und 5'-ICI CIC ICI CIC ICI CIC ICI CIC IC-3' ist, zur Verwendung als Impfstoff gegen *M. tuberculosis* bei einem Menschen.

## Revendications

1. Composition immunogène comprenant :
a) une protéine de fusion de la protéine TB 10.4 et d'une protéine du complexe Ag85, et
b) un adjuvant comprenant au moins un peptide polycationique et au moins un oligonucléotide qui est oligo dIC,
dans laquelle la protéine du complexe Ag85 est une protéine Ag85B, la quantité totale de la protéine TB 10.4 et de la protéine Ag85B est comprise entre 5,0 et 25,0 µg, et l'adjuvant est un mélange de NH₂-KLKLLLLLKLK-COOH et de 5'-ICI CIC ICI CIC ICI CIC ICI CIC IC-3', pour utilisation comme vaccin contre *M. tuberculosis* chez l'homme.
